# EUROPEAN PATENT SPECIFICATION

(11) **EP 3 871 724 B1**
(45) Date of publication and mention of the grant of the patent: **13.03.2024**
(21) Application number: 21159678.8
(22) Date of filing: 26.02.2021
(51) Int. Cl.: A61M 16/08

(54) **AN OUTLET FITTING FOR A POSITIVE EXPIRATORY THERAPY DEVICE**
AUSLASSANSCHLUSS FÜR EINE POSITIVE EXSPIRATORISCHE THERAPIEVORRICHTUNG
RACCORD DE SORTIE POUR UN DISPOSITIF DE THÉRAPIE EXPIRATOIRE POSITIVE

(30) Priority: 27.02.2020 GB 202002799
(43) Date of publication of application: 01.09.2021
(73) Proprietor: Intersurgical AG, 9490 Vaduz (LI)
(72) Inventor: MIN, Yoon, Wokingham, Berkshire RG41 2RZ (GB)
(74) Representative: Adamson Jones

(56) References cited:
- FR-A1- 2 899 483
- FR-B1- 2 899 483
- US-A- 6 135 108
- US-A1- 2004 040 557
- US-A1- 2009 241 963
- US-A1- 2018 094 730

## Description

In the human body, for adequate lung function to occur, the alveoli fill and discharge exchanging nutrients with the bloodstream. The alveoli can be constrained by obstructions or trapped air, leading to decreased function, and illnesses such as chronic obstructive pulmonary disease (COPD), cystic fibrosis, asthma, amongst other pulmonary conditions.

Positive Expiratory Pressure (PEP) therapy uses intermittent pressure to clear such obstructions into larger pathways so that a patient can expel them. This relief of pressure occurs normally in healthy patients, but may require specialized devices for compromised individuals. In such devices, the intermittent pressure added during exhalation drives air into the alveoli, forcing out the obstructions and directing the undesirable elements to the larger airways so that they can be coughed out by the patient.

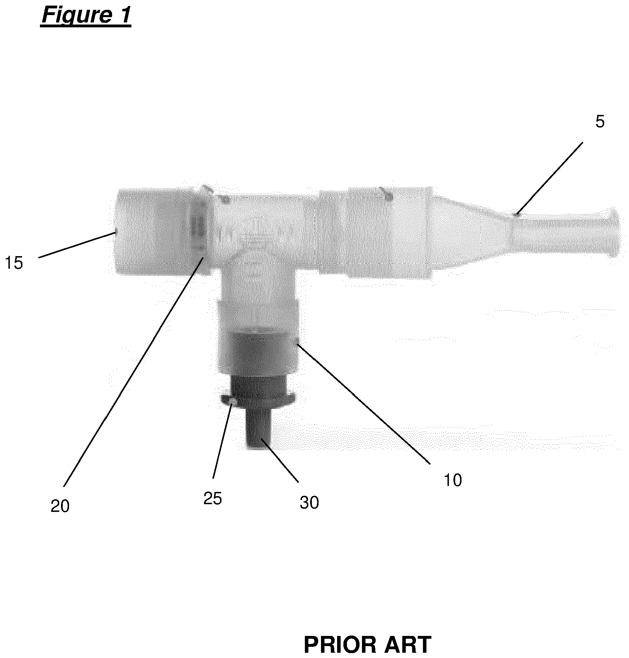

An example of such a device is the PEP device illustrated in Figure 1. The device includes a mouthpiece 5 for the patient to breathe into the device, an expiratory port 10 for expelling exhaled air into the atmosphere and an inspiratory port 15 for inhaling air from the atmosphere. The inspiratory port 15 includes a one-way inspiratory valve 20 such that in use, air can flow into the device via the inspiratory port 15 during inhalation, but must exit the device via the expiratory port 10 during exhalation.

Inserted within the expiratory port 10 is an airflow resistor 25 having a spigot 30. The spigot 30 is specifically dimensioned so that the resistor 25 creates a desired predetermined resistance against the exhalation breath of the patient. The resistor 25 is selectively interchangeable with other similar resistors having spigots of different dimensions, so that the resistance may be varied dependent on the needs of the patient.

In order to allow exhalation, the spigot 30 must be open-ended and have a form similar to that of the spigots of other respiratory apparatus, to which tubing is connected. This nature of the spigot can often lead to operators (who may be rushed and/or distracted), or patients (who may be less informed), mistakenly assuming that the spigot is for connection to another component, for example an oxygen tube. Such misconnection prevents the device from functioning correctly and can be dangerous. Since the spigot is placed at the expiratory port of the device, misconnection may alter the resistance created by the resistor, and indeed may block the port, preventing exhalation entirely, or even cause undesirable gas/surfactant to be input into the device, and hence the patient's airways.

A similar problem is solved in relation to the inspiratory port 15 by making the inspiratory port 15 a non-standard connector, preventing misconnection of standard breathing tubes. However, since the expiratory port 10 needs to receive a resistor, and the spigot 30 needs to be open-ended and dimensioned very specifically to create the desired resistance, this is not possible at the expiratory port.

One other possible solution would be to exchange the spigot for a hole in the resistor, so as to prevent misleading the patient or operator, whilst still allowing exhalation. However, the holes in the resistors would require a smaller diameter than the corresponding spigot in order to create an equivalent resistance through the device. Since the spigot diameters are small, generally ranging from around 1.5mm to 6mm, accurately manufacturing such holes is particularly difficult, making this solution undesirable.

US2004/040557 discloses a device for inspiration of a mist from a nebulizer, the device comprising an inlet connector adapted to engage an outlet port of a nebulizer. US6135108 discloses an apparatus for preventing blockage or occlusion of a fluid flow path, such as a flow path through which a patient's exhalation gas flows. US2009/241963 discloses a suction arrangement that includes a suction source with a single inlet and a connector of caltrop shape with four interconnected ribbed ports, one of which is connected to the suction inlet.

There is provided an outlet fitting for a positive expiratory therapy device which overcomes or substantially mitigates the abovementioned and/or other disadvantages associated with the prior art.

According to a first aspect of the invention there is provided an outlet fitting for a positive expiratory therapy device, the outlet fitting comprising:
a first hollow arm having a tubular connector arranged for engagement with an expiratory port of the positive expiratory therapy device, the first hollow arm having an inlet end for receiving exhaled air from the positive expiratory therapy device and an outlet end for expelling the exhaled air, the tubular connector being at the inlet end; and
a second hollow arm in fluid communication with the first hollow arm, the second hollow arm having an inlet end for receiving the exhaled air from the outlet end of the first hollow arm and an outlet end for expelling the exhaled air into the atmosphere;
the outlet fitting having a minimum cross-sectional area of fluid flow that is less than a cross-sectional area of fluid flow at the expiratory port, and the second hollow arm having a side surface and at least one protrusion extending from the side surface at or to the outlet end of the second hollow arm, such that connection of a tube to the outlet end of the second hollow arm is impeded,
wherein the at least one protrusion extends from the inlet end of the second hollow arm to the outlet end of the second hollow arm and ends at the outlet end of the second hollow arm,
wherein the at least one protrusion comprises one or more side surfaces which each extend from an edge on the side surface of the second hollow arm, and
wherein the width of the one or more side surfaces of the at least one protrusion is reduced as the protrusion extends from the inlet end of the second hollow arm to the outlet end of the second hollow arm.

This outlet fitting is advantageous in that it impedes the connection of a tube to the outlet end of the second hollow arm, and in that connection to the second hollow arm is impeded across the entire length of the second hollow arm.

One or both of the first and second hollow arms may be substantially cylindrical in shape. The side surface of the second hollow arm may be a curved surface, ie a substantially cylindrical side surface. The first and second hollow arms may have central longitudinal axes that are substantially parallel or aligned. The side surface of the second hollow arm may extend from the inlet end of the second hollow arm to the outlet end of the second hollow arm.

The one or more side surfaces of the at least one protrusion may be two opposing side surfaces, which may each extend from an edge on the side surface of the second hollow arm. The edges from which the opposing side surfaces extend may be substantially linear, may be substantially parallel to the central longitudinal axis of the second hollow arm, and may extend along at least the majority of the length of the second hollow arm.

The side surface of the at least one protrusion may be angled relative to the side surface of the hollow arm at an angle of at least 45 degrees, at least 60 degrees, at least 75 degrees or at least 90 degrees. This may be advantageous in that conventional tubes are cylindrical, and thus would not readily fit over the at least one protrusion of the second hollow arm, at least not whilst maintaining an air-tight fit, thus indicating to an operator that the outlet end of the second hollow arm is not intended for connection.

The at least one protrusion may have a global form that is substantially planar. The depth of the at least one protrusion may be less than the width and the length of the at least one protrusion, and the depth may be substantially uniform across the full extent of the at least one protrusion. The at least one protrusion may extend from the side surface of the second hollow arm substantially in a plane. The at least one protrusion may have a central plane, and a substantially uniform depth to each side of the central plane.

Where the side surface of the second hollow arm is a substantially cylindrical side surface, the central plane of the at least one protrusion may extend from the substantially cylindrical side surface substantially perpendicularly to a tangential central plane of the substantially cylindrical side surface. The central plane of the at least one protrusion may extend from the substantially cylindrical side surface substantially parallel to a normal vector of the substantially cylindrical side surface. The side surface(s) of the at least one protrusion may be substantially perpendicular to a tangential plane of the substantially cylindrical side surface. The side surface(s) of the at least one protrusion may be substantially parallel to a normal vector of the substantially cylindrical surface.

Substantially perpendicular may comprise, for example, at an angle of between 60 and 120 degrees, or at an angle of between 75 and 105 degrees. Substantially parallel may comprise, for example, at an angle of up to 30 degrees, or at angle of up to 15 degrees.

The at least one protrusion may have a cross-sectional shape, eg in the central plane described above, that is triangular, trapezoidal, or arcuate in shape. The at least one protrusion may have a radially outer surface that is inclined relative to a longitudinal axis of the second hollow arm, eg the radially outer surface may have a longitudinal axis, eg a central longitudinal axis, that is at an angle of greater than 0 degrees, greater than 15 degrees or greater than 25 degrees, relative to a longitudinal axis, eg a central longitudinal axis, of the second hollow arm.

The at least one protrusion may have a length greater than its width, where the length is defined as the dimension being parallel to the central longitudinal axis of the second hollow arm, and the width is defined as being perpendicular to the central longitudinal axis of the second hollow arm.

Optionally, the at least one protrusion may have an end surface that extends between the radially outer surface of the at least one protrusion and the second hollow arm. The end surface may extend substantially perpendicularly to the tangential plane of the side surface of the second hollow arm from which the at least one protrusion extends, and to the side surfaces of the at least one protrusion. The end surface may extend from the side surface of the second hollow arm at the outlet end.

The width of the at least one protrusion, eg its dimension in a radial direction, may vary across its length, ie across its axial extent. The side surface(s) of the at least one protrusion may be a tapered surface.

Where the side surface of the second hollow arm is a substantially cylindrical side surface, the radially outer surface of the at least one protrusion may extend from an outlet end of the second hollow arm at a position that is radially closer to the substantially cylindrical side surface than the position of the radially outer surface at an inlet end of the second hollow arm.

The at least one protrusion may therefore provide a radially outer surface that is inclined towards, and hence faces towards, the outlet end of the second hollow arm, and hence the end at which tubing may be mistakenly engaged. This may be advantageous in that conventional tubing is cylindrical, and thus would not fit comfortably over the at least one protrusion of the second hollow arm, at least not whilst maintaining an air-tight fit, thus indicating to an operator that the outlet end of the second hollow arm is not intended for connection. Furthermore, the inclined radially outer surface may reduce the risk of frictional engagement between tubing and the at least one protrusion. The taper of the at least one protrusion may be substantially continuous, such that the radially outer surface is smooth and/or the radially outer surface may be substantially flat.

The at least one protrusion may comprise more than one protrusion, for example, two, three or four protrusions. Where the at least one protrusion comprises more than one protrusion, the protrusions may be positioned symmetrically about the central longitudinal axis of the second hollow arm. The protrusions may be positioned symmetrically about the central longitudinal axis of the second hollow arm when viewed from the end of the second hollow arm, ie when viewed along the central longitudinal axis. Where there are two protrusions, there may be a 180-degree angle about the cylindrical surface that separates the two protrusions.

Where there are three protrusions, there may be a 120-degree angle about the cylindrical surface that separates any two protrusions. Where there are four protrusions, there may be a 90-degree angle about the cylindrical surface that separates any two protrusions.

Where the at least one protrusion comprises more than one protrusion, the protrusions may have the same shape and/or size. Where the at least one protrusion comprises two protrusions, the protrusions may be symmetrical about the central longitudinal axis of the second hollow arm. Where the at least one protrusion comprises four protrusions, alternate protrusions may be symmetrical about the central longitudinal axis of the second hollow arm.

Where the at least one protrusion comprises more than one protrusion, the protrusions may be arranged at the same position along the length of the side surface of the second hollow arm. The side surface(s) of each protrusion may extend from the side surface of the second hollow arm at the same position along the length of the side surface of the second hollow arm, ie the end surface of each protrusion may be the same distance from the inlet end and/or the outlet end of the second hollow arm.

The first hollow arm may have an internal diameter and an external diameter. The second hollow arm may have an internal diameter and an external diameter. The internal diameter of the second hollow arm at the outlet end may be less than the internal diameter of the first hollow arm at the inlet end. The second hollow arm may have an internal diameter, at least at the outlet end, of between approximately 1.5mm and approximately 6.0mm, for example 1.5mm, 3.5mm or 6.0mm.

The internal diameter of one or both of the first and second hollow arms may be substantially constant along their length. The external diameter of one or both of the first and second hollow arms may be substantially constant along their length. The change in internal diameter between the first hollow arm and the second hollow arm may be a step change, ie a shoulder may be formed internally between the first and second hollow arms. The external diameter being substantially constant may comprise, for example, an external diameter that varies by up to 10%, up to 5%, or up to 2.5% along its length.

Alternatively, the internal diameter of the first hollow arm and/or the second hollow arm may vary such that the internal progression between the first and second hollow arms is continuous. Similarly, the external diameter of the first hollow arm and/or the second hollow arm may vary such that the first and second hollow arms form first and second portions of a single hollow arm.

Typically, expiratory ports of positive expiratory therapy devices are conventional tubular ports, arranged for engagement with tubular connectors. The tubular connector of the outlet fitting may have an engagement surface arranged for engagement with the expiratory port of the positive expiratory therapy device. The engagement surface may be an external surface of the tubular connector that engages with an internal surface of the expiratory port, ie the tubular connector may be a male tubular connector. The first hollow arm, at least at the tubular connector, may therefore have an external diameter that is substantially equal to an internal diameter of the expiratory port, so as to have a frictional fit with the expiratory port. The first hollow arm, at least at the tubular connector, may also have a cross-sectional area defined by the external surface that is substantially equal to the cross-sectional area defined by the internal surface of the expiratory port, so as to have a frictional fit with the expiratory port. Hence, the outlet fitting may have a minimum cross-sectional area of fluid flow that is less than the cross-sectional area defined by the external surface of the tubular connector of the first hollow arm.

The width of the at least one protrusion, eg its dimension in a radial direction, at its narrowest point may be approximately equal to the internal diameter of the second hollow arm at the outlet end. The width of the at least one protrusion, eg its dimension in a radial direction, at its narrowest point may be between 0.5 times and 1.7 times the internal diameter of the second hollow arm at the outlet end. The width of the at least one protrusion at its narrowest point may be between 1 mm and 5mm, between 2mm and 4mm, between 2.5 and 3.5mm, or between 3 and 3.5mm, for example approximately 3.25mm. The width of the at least one protrusion at its widest point may be approximately equal to the external diameter of the second hollow arm at the outlet end. The width of the at least one protrusion at its widest point may be between 0.5 times and 1.3 times the external diameter of the second hollow arm at the outlet end. The at least one protrusion may increase the external diameter of the outlet fitting at the outlet end of the second hollow arm to be between approximately two times and approximately five times the internal diameter of the second hollow arm at the outlet end. This may be advantageous in that it gives a clear and obvious indication that the outlet end of the second hollow arm is not intended for connection.

The at least one protrusion may increase the external diameter of the outlet fitting at the outlet end of the second hollow arm to be greater than the internal diameter of conventional oxygen supply tubing, which is typically around 4mm. This may be advantageous in that it impedes or prevents a conventional oxygen supply tube from being placed over the outlet end of the second hollow arm.

The outlet fitting may further comprise a flange. The flange may be a circumferential flange, which may be orientated perpendicularly relative to the first and/or second hollow arms, eg such that the flange extends radially from the first and/or second hollow arms. The flange may be positioned between the first and second hollow arms. The flange may extend further radially outwardly than the first and/or second hollow arms, eg to form a shoulder on the inlet/outlet side of the outlet fitting. This may be advantageous in that it allows a patient or operator to apply force against the flange when engaging the outlet fitting with the expiratory port of a positive expiratory therapy device, and may provide a stop that abuts the end of the expiratory port. The at least one protrusion may abut the flange.

The tubular connector may be substantially cylindrical in shape. The tubular connector may have an external diameter that is smaller than the internal diameter of the expiratory port of the conventional positive expiratory therapy device so as to be received therein during use. The external diameter of the first hollow arm at the inlet end may be smaller than the internal diameter of the expiratory port of the conventional positive expiratory therapy device so as to be received therein during use. The tubular connector may be arranged to engage with the expiratory port of the positive expiratory therapy device via a frictional engagement, a threaded engagement, or any other conventional engagement.

The positive expiratory therapy device may be a handheld device. The outlet fitting may be made from a plastic, for example a thermoplastic such as polypropylene. The outlet fitting may be manufactured by any conventional method for forming plastic materials, such as injection moulding.

According to a second aspect of the invention there is provided a system comprising a positive expiratory therapy device and an outlet fitting according to the first aspect of the invention.

According to a third aspect of the invention there is provided a positive expiratory therapy device comprising an outlet fitting according to the first aspect of the invention.

According to fourth aspect not in accordance with the claimed invention, there is provided a positive expiratory therapy device having a patient interface in fluid communication with both an inspiratory port and an expiratory port, the positive expiratory therapy device further comprising an outlet fitting, the outlet fitting comprising:
a first hollow arm arranged for engagement with the expiratory port of the positive expiratory therapy device, the first hollow arm having an inlet end for receiving exhaled air from the positive expiratory therapy device and an outlet end for expelling the exhaled air; and
a second hollow arm in fluid communication with the first hollow arm, the second hollow arm having an inlet end for receiving the exhaled air from the outlet end of the first hollow arm and an outlet end for expelling the exhaled air into the atmosphere;
the outlet fitting having a minimum cross-sectional area of fluid flow that is less than a cross-sectional area of fluid flow at the expiratory port, and the second hollow arm having a side surface and at least one protrusion extending from the side surface at or to the outlet end, such that connection of a tube to the outlet end of the second hollow arm is impeded.

The patient interface may be arranged such that a patient can breathe into the device in use. The patient interface may be a mouthpiece or a mask. The expiratory port may be for expelling exhaled air into the atmosphere. The inspiratory port may be for inhaling air from the atmosphere. The positive expiratory therapy device may further comprise a one-way valve positioned between the inspiratory port and the patient interface. The one-way valve may be arranged such that in use, air can flow from the inspiratory port to the patient interface, but not from the patient interface to the inspiratory port.

The positive expiratory therapy device may be a handheld device. The handheld device may be a generally T-shaped device. The outlet fitting may be integrated with the positive expiratory therapy device, eg as part of the same moulded component as the expiratory port, or may alternatively be a separate component from the positive expiratory therapy device. Where the outlet fitting is a separate component from the positive expiratory therapy device, the outlet fitting may be removable and/or replaceable.

Practicable embodiments of the invention will now be described, by way of example only, with reference to the accompanying drawings, of which:
Figure 1 is a PEP device known in the art; and
Figure 2 is a perspective view of an outlet fitting 100 according to the invention; and
Figure 3 is a cross-section through the line X-X of the outlet fitting 100 of Figure 2.

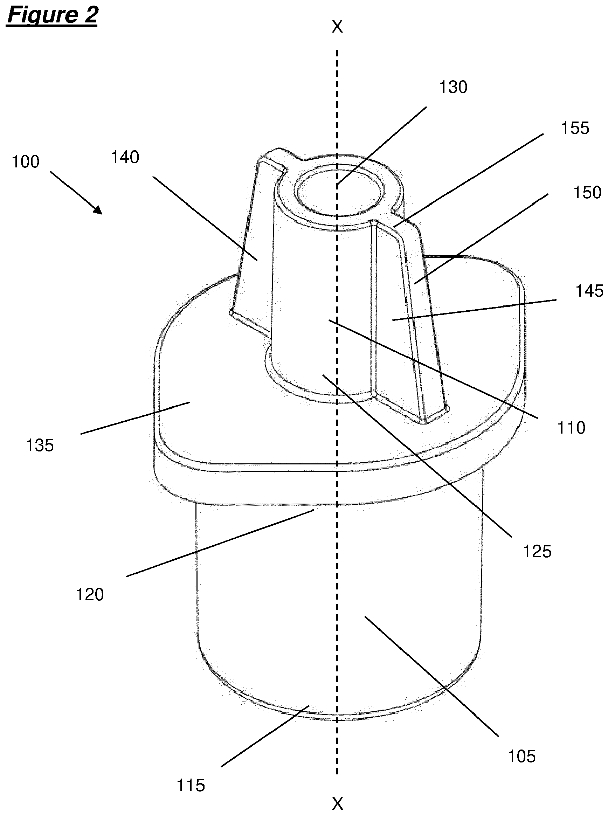

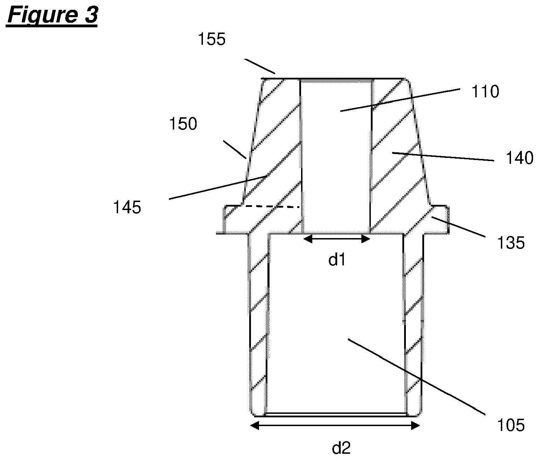

Figures 2 and 3 illustrate an outlet fitting 100 according to the invention. The outlet fitting 100 comprises a first hollow arm 105 and a second hollow arm 110 in fluid communication with one another.

The first hollow arm 105 comprises a tubular connector arranged for engagement with an expiratory port of a positive expiratory device in use, such as the expiratory port 10 of the positive expiratory device of Figure 1. The first hollow arm 105 further comprises an inlet end 115 arranged to receive exhaled air from the positive expiratory therapy device in use, and an outlet end 120 for expelling the exhaled air from the first hollow arm 105 into the second hollow arm 110. Thus, the tubular connector is positioned at the inlet end 115 of the first hollow arm 105.

The second hollow arm 110 comprises an inlet end 125 for receiving the expelled air from the first hollow arm 105, and an outlet end 130 for expelling that air from the second hollow arm 110 into the atmosphere.

Positioned between the first and second hollow arms 105, 110 on the external surface of the outlet fitting 100 is a flange 135 which extends outwardly between the outlet end 120 of the first hollow arm 105 and the inlet end 125 of the second hollow arm 110.

The first and second hollow arms 105, 110 are both substantially cylindrical hollow arms, each having an internal diameter and an external diameter that are substantially constant along their length. The internal and external diameters of the first hollow arm 105 are greater than the internal and external diameters of the second hollow arm 110 respectively. However, it will be appreciated that the internal and/or external diameter of the first hollow arm 105 and/or the second hollow arm 110 may vary along their length, provided the minimum internal diameter d1 of the outlet fitting 100 is less than the internal diameter of the expiratory port, which in this example corresponds to the external diameter d2 of the first hollow arm 105 at the inlet end 115, so as to provide a resistance to air flow (ie breathing) through the positive expiratory therapy device in use.

Two protrusions 140 extend from the cylindrical side surface of the second hollow arm 110. In this embodiment, the protrusions 140 extend from the inlet end 125 of the side surface to the outlet end 130 of the side surface, however it is anticipated that the protrusions 140 would perform their intended function provided they at least extend at or to the outlet end 130 of the second outlet arm 110.

The protrusions 140 are positioned symmetrically about a central longitudinal axis of the second hollow arm 110. That is, the protrusions 140 extend from the side surface at the same position relative to the inlet and outlet ends 125, 130 of the second hollow arm 110, and extend in the same plane as one another, ie there is a 180 degree angle about the cylindrical surface that separates the two protrusions 140. The shape of the protrusions 140 is also symmetrical about the central longitudinal axis of the second hollow arm 110. That is, the protrusions 140 have the same shape but are a mirror image of one another owing to their 180-degree separation about the cylindrical surface.

The protrusions 140 are generally flat. That is, they reside in a single plane. The protrusions 140 comprise a pair of opposing side surfaces 145 that are separated by a radially outer surface 150 and an end surface 155. The protrusions 140 have a trapezoidal cross-sectional shape, and are generally wing-shaped.

The protrusions 140 have a length greater than their width. The radially outer surface 150 is inclined relative to the longitudinal axis of the second hollow arm 110 such that the width of the protrusions 140 varies across their length. The width of the side surfaces 145 reduces as the protrusions 140 extend from the inlet end 125 of the second hollow arm 110 to the outlet end 130 of the second hollow arm 110. The radially outer surface 150 is a flat surface, and thus the taper of the side surfaces 145 is continuous.

In alternative embodiments, it is anticipated that the protrusions 140 may have different forms and/or dimensions, provided they impede an operator from engaging a tube with the outlet end 130 of the second hollow arm 110. It is also anticipated that the outlet fitting 100 may instead be integrated with a PEP therapy device, ie preassembled within the device, rather than being insertable into the expiratory port of such a device.

## Claims

1. An outlet fitting (100) for a positive expiratory therapy device, the outlet fitting comprising:
a first hollow arm (105) having a tubular connector arranged for engagement with an expiratory port of the positive expiratory therapy device, the first hollow arm (105) having an inlet end (115) for receiving exhaled air from the positive expiratory therapy device and an outlet end (120) for expelling the exhaled air, the tubular connector being at the inlet end (115);
a second hollow arm (110) in fluid communication with the first hollow arm (105), the second hollow arm (110) having an inlet end (125) for receiving the exhaled air from the outlet end of the first hollow arm (105) and an outlet end (130) for expelling the exhaled air into the atmosphere;
the outlet fitting (100) having a minimum cross-sectional area of fluid flow that is less than a cross-sectional area of fluid flow at the expiratory port;
the second hollow arm (110) having a side surface and at least one protrusion (140) extending from the side surface at or to the outlet end (130) of the second hollow arm (110), such that connection of a tube to the outlet end (130) of the second hollow arm (110) is impeded,
wherein the at least one protrusion (140) extends from the inlet end (125) of the second hollow arm (110) to the outlet end (130) of the second hollow arm (110) and ends at the outlet end (130) of the second hollow arm (110),
wherein the at least one protrusion (140) comprises one or more side surface (145) which each extend from an edge on the side surface of the second hollow arm (110), and
wherein the width of the one or more side surface (145) of the at least one protrusion (140) is reduced as the protrusion (140) extends from the inlet end (125) of the second hollow arm (110) to the outlet end (130) of the second hollow arm (110).

2. An outlet fitting (100) according to Claim 1, wherein the side surface of the second hollow arm (110) is a substantially cylindrical side surface, and wherein the at least one protrusion (140) has a central plane and the central plane of the at least one protrusion (140) extends from the substantially cylindrical side surface substantially parallel to a normal vector of the substantially cylindrical side surface.

3. An outlet fitting (100) according to any preceding claim, wherein the one or more side surface (145) of the at least one protrusion (140) is angled relative to the side surface of the second hollow arm at angle of at least 45 degrees, at least 60 degrees, at least 75 degrees or at least 90 degrees.

4. An outlet fitting (100) according to any preceding claim, wherein the at least one protrusion (140) has a global form that is substantially planar.

5. An outlet fitting (100) according to any preceding claim, wherein the at least one protrusion (140) comprises more than one protrusion, and the protrusions are positioned symmetrically about the central longitudinal axis of the second hollow arm (110).

6. An outlet fitting (100) according to any preceding claim, wherein the at least one protrusion (140) has a cross-sectional shape that is substantially triangular or trapezoidal in shape.

7. An outlet fitting (100) according to any preceding claim, wherein the at least one protrusion (140) has a radially outer surface (150) that is inclined at an angle of greater than 15 degrees relative to a longitudinal axis of the second hollow arm (110).

8. An outlet fitting (100) according to any preceding claim, wherein the width of the at least one protrusion (140) varies across its length.

9. An outlet fitting according to any preceding claim, wherein the at least one side surface (145) is a tapered surface.

10. An outlet fitting according to Claim 9, when dependent on Claim 7, wherein the taper is substantially continuous, such that the radially outer surface (150) of the at least one protrusion (140) is smooth and/or substantially flat.

11. An outlet fitting (100) according to Claim 9 or Claim 10, wherein the radially outer surface (150) of the at least one protrusion (140) extends from the outlet end (130) of the second hollow arm (110) at a position that is radially closer to the substantially cylindrical side surface than the position of the radially outer surface (150) at the inlet end (125) of the second hollow arm (110).

12. An outlet fitting (100) according to any preceding claim, wherein the at least one protrusion (140) provides a radially outer surface (150) that is inclined towards, and hence faces towards, the outlet end (130) of the second hollow arm (110).

13. A system comprising a positive expiratory therapy device and an outlet fitting (100) according to any one of Claims 1 to 12.

14. A positive expiratory therapy device comprising the outlet fitting (100) according to any one of claims 1-12.

15. A positive expiratory therapy device according to Claim 14, wherein the outlet fitting (100) is integrated with the positive expiratory therapy device.

## Patentansprüche

1. Auslassanschluss (100) für eine positive exspiratorische Therapievorrichtung, wobei der Auslassanschluss Folgendes umfasst:
einen ersten hohlen Arm (105), der einen rohrförmigen Verbinder aufweist, der für einen Eingriff mit einer exspiratorischen Öffnung der positiven exspiratorischen Therapievorrichtung angeordnet ist, wobei der erste hohle Arm (105) ein Einlassende (115) zum Aufnehmen ausgeatmeter Luft von der positiven exspiratorischen Therapievorrichtung und ein Auslassende (120) zum Ausstoßen der ausgeatmeten Luft aufweist, wobei sich der rohrförmige Verbinder an dem Einlassende (115) befindet;
einen zweiten hohlen Arm (110), der in Fluidkommunikation mit dem ersten hohlen Arm (105) steht, wobei der zweite hohle Arm (110) ein Einlassende (125) zum Aufnehmen der ausgeatmeten Luft von dem Auslassende des ersten hohlen Arms (105) und ein Auslassende (130) zum Ausstoßen der ausgeatmeten Luft in die Atmosphäre aufweist;
wobei der Auslassanschluss (100) eine minimale Querschnittsfläche der Fluidströmung aufweist, die kleiner ist als eine Querschnittsfläche der Fluidströmung an der exspiratorischen Öffnung;
wobei der zweite hohle Arm (110) eine Seitenoberfläche und mindestens einen Vorsprung (140) aufweist, der sich derart von der Seitenoberfläche an oder zu dem Auslassende (130) des zweiten hohlen Arms (110) erstreckt, dass eine Verbindung eines Rohrs mit dem Auslassende (130) des zweiten hohlen Arms (110) behindert wird,
wobei sich der mindestens eine Vorsprung (140) von dem Einlassende (125) des zweiten hohlen Arms (110) zu dem Auslassende (130) des zweiten hohlen Arms (110) erstreckt und an dem Auslassende (130) des zweiten hohlen Arms (110) endet,
wobei der mindestens eine Vorsprung (140) eine oder mehrere Seitenoberflächen (145) umfasst, die sich jeweils von einer Kante an der Seitenoberfläche des zweiten hohlen Arms (110) erstrecken, und
wobei die Breite der einen oder der mehreren Seitenoberflächen (145) des mindestens einen Vorsprungs (140) reduziert wird, wenn sich der Vorsprung (140) von dem Einlassende (125) des zweiten hohlen Arms (110) zu dem Auslassende (130) des zweiten hohlen Arms (110) erstreckt.

2. Auslassanschluss (100) nach Anspruch 1, wobei die Seitenoberfläche des zweiten hohlen Arms (110) eine im Wesentlichen zylindrische Seitenoberfläche ist und wobei der mindestens eine Vorsprung (140) eine Mittelebene aufweist und sich die Mittelebene des mindestens einen Vorsprungs (140) von der im Wesentlichen zylindrischen Seitenoberfläche im Wesentlichen parallel zu einem Normalenvektor der im Wesentlichen zylindrischen Seitenoberfläche erstreckt.

3. Auslassanschluss (100) nach einem vorhergehenden Anspruch, wobei die eine oder die mehreren Seitenoberflächen (145) des mindestens einen Vorsprungs (140) relativ zu der Seitenoberfläche des zweiten hohlen Arms in einem Winkel von mindestens 45 Grad, mindestens 60 Grad, mindestens 75 Grad oder mindestens 90 Grad abgewinkelt sind.

4. Auslassanschluss (100) nach einem vorhergehenden Anspruch, wobei der mindestens eine Vorsprung (140) eine globale Form aufweist, die im Wesentlichen planar ist.

5. Auslassanschluss (100) nach einem vorhergehenden Anspruch, wobei der mindestens eine Vorsprung (140) mehr als einen Vorsprung umfasst und die Vorsprünge symmetrisch um die Mittellängsachse des zweiten hohlen Arms (110) positioniert sind.

6. Auslassanschluss (100) nach einem vorhergehenden Anspruch, wobei der mindestens eine Vorsprung (140) eine Querschnittsform aufweist, die eine im Wesentlichen dreieckige oder trapezförmige Form hat.

7. Auslassanschluss (100) nach einem vorhergehenden Anspruch, wobei der mindestens eine Vorsprung (140) eine radial äußere Oberfläche (150) aufweist, die in einem Winkel von mehr als 15 Grad relativ zu einer Längsachse des zweiten hohlen Arms (110) geneigt ist.

8. Auslassanschluss (100) nach einem vorhergehenden Anspruch, wobei die Breite des mindestens einen Vorsprungs (140) über seine Länge variiert.

9. Auslassanschluss nach einem vorhergehenden Anspruch, wobei die mindestens eine Seitenoberfläche (145) eine verjüngte Oberfläche ist.

10. Auslassanschluss nach Anspruch 9, wenn abhängig von Anspruch 7, wobei die Verjüngung im Wesentlichen kontinuierlich ist, sodass die radial äußere Oberfläche (150) des mindestens einen Vorsprungs (140) glatt und/oder im Wesentlichen flach ist.

11. Auslassanschluss (100) nach Anspruch 9 oder Anspruch 10, wobei sich die radial äußere Oberfläche (150) des mindestens einen Vorsprungs (140) an einer Position, die radial näher an der im Wesentlichen zylindrischen Seitenoberfläche liegt als die Position der radial äußeren Oberfläche (150) an dem Einlassende (125) des zweiten hohlen Arms (110), von dem Auslassende (130) des zweiten hohlen Arms (110) erstreckt.

12. Auslassanschluss (100) nach einem vorhergehenden Anspruch, wobei der mindestens eine Vorsprung (140) eine radial äußere Oberfläche (150) bereitstellt, die in Richtung des Auslassendes (130) des zweiten hohlen Arms (110) geneigt ist und daher diesem zugewandt ist.

13. System, umfassend eine positive exspiratorische Therapievorrichtung und einen Auslassanschluss (100) nach einem der Ansprüche 1 bis 12.

14. Positive exspiratorische Therapievorrichtung, umfassend den Auslassanschluss (100) nach einem der Ansprüche 1-12.

15. Positive exspiratorische Therapievorrichtung nach Anspruch 14, wobei der Auslassanschluss (100) in die positive exspiratorische Therapievorrichtung integriert ist.

## Revendications

1. Raccord de sortie (100) pour un dispositif de thérapie expiratoire positive, le raccord de sortie comprenant :
un premier bras creux (105) comportant un connecteur tubulaire agencé pour venir en prise avec un orifice expiratoire du dispositif de thérapie expiratoire positive, le premier bras creux (105) comportant une extrémité d'entrée (115) destinée à recevoir l'air expiré provenant du dispositif de thérapie expiratoire positive et une extrémité de sortie (120) destinée à expulser l'air expiré, le connecteur tubulaire se trouvant au niveau de l'extrémité d'entrée (115) ;
un second bras creux (110) en communication fluidique avec le premier bras creux (105), le second bras creux (110) comportant une extrémité d'entrée (125) destinée à recevoir l'air expiré depuis l'extrémité de sortie du premier bras creux (105) et une extrémité de sortie (130) destinée à expulser l'air expiré dans l'atmosphère ;
le raccord de sortie (100) comportant une surface transversale minimale d'écoulement de fluide qui est moindre qu'une surface transversale d'écoulement de fluide au niveau de l'orifice expiratoire ;
le second bras creux (110) comportant une surface latérale et au moins une saillie (140) s'étendant depuis la surface latérale au niveau de l'extrémité de sortie (130) du second bras creux (110) ou jusqu'à celle-ci, de sorte que le raccordement d'un tube à l'extrémité de sortie (130) du second bras creux (110) soit entravé,
ladite au moins une saillie (140) s'étendant depuis l'extrémité d'entrée (125) du second bras creux (110) jusqu'à l'extrémité de sortie (130) du second bras creux (110) et se terminant au niveau de l'extrémité de sortie (130) du second bras creux (110), ladite au moins une saillie (140) comprenant une ou plusieurs surfaces latérales (145) qui s'étendent chacune à partir d'un bord sur la surface latérale du second bras creux (110), et
la largeur desdites une ou plusieurs surfaces latérales (145) de ladite au moins une saillie (140) étant réduite à mesure que la saillie (140) s'étend depuis l'extrémité d'entrée (125) du second bras creux (110) jusqu'à l'extrémité de sortie (130) du second bras creux (110).

2. Raccord de sortie (100) selon la revendication 1, ladite surface latérale du second bras creux (110) étant une surface latérale sensiblement cylindrique, et ladite au moins une saillie (140) comportant un plan central et ledit plan central de ladite au moins une saillie (140) s'étendant depuis la surface latérale sensiblement cylindrique sensiblement parallèlement à un vecteur normal de la surface latérale sensiblement cylindrique.

3. Raccord de sortie (100) selon l'une quelconque des revendications précédentes, lesdites une ou plusieurs surfaces latérales (145) de ladite au moins une saillie (140) étant inclinées par rapport à la surface latérale du second bras creux suivant un angle d'au moins 45 degrés, d'au moins 60 degrés, d'au moins 75 degrés ou d'au moins 90 degrés.

4. Raccord de sortie (100) selon l'une quelconque des revendications précédentes, ladite au moins une saillie (140) comportant une forme globale qui est sensiblement plane.

5. Raccord de sortie (100) selon l'une quelconque des revendications précédentes, ladite au moins une saillie (140) comprenant plus d'une saillie, et lesdites saillies étant positionnées symétriquement autour de l'axe longitudinal central du second bras creux (110).

6. Raccord de sortie (100) selon l'une quelconque des revendications précédentes, ladite au moins une saillie (140) comportant une forme transversale qui est sensiblement de forme triangulaire ou trapézoïdale.

7. Raccord de sortie (100) selon l'une quelconque des revendications précédentes, ladite au moins une saillie (140) comportant une surface radialement externe (150) qui est inclinée suivant un angle supérieur à 15 degrés par rapport à l'axe longitudinal du second bras creux (110).

8. Raccord de sortie (100) selon l'une quelconque des revendications précédentes, ladite largeur de ladite au moins une saillie (140) variant sur sa longueur.

9. Raccord de sortie selon l'une quelconque des revendications précédentes, ladite au moins une surface latérale (145) étant une surface conique.

10. Raccord de sortie selon la revendication 9, lorsqu'elle dépend de la revendication 7, ledit cône étant sensiblement continu, de sorte que la surface radialement externe (150) de ladite au moins une saillie (140) soit lisse et/ou sensiblement plate.

11. Raccord de sortie (100) selon la revendication 9 ou la revendication 10, ladite surface radialement externe (150) de ladite au moins une saillie (140) s'étendant depuis l'extrémité de sortie (130) du second bras creux (110) au niveau d'une position qui est radialement plus proche de la surface latérale sensiblement cylindrique que la position de la surface radialement externe (150) au niveau de l'extrémité d'entrée (125) du second bras creux (110).

12. Raccord de sortie (100) selon l'une quelconque des revendications précédentes, ladite au moins une saillie (140) fournissant une surface radialement externe (150) qui est inclinée en direction, et donc tournée en direction, de l'extrémité de sortie (130) du second bras creux (110).

13. Système comprenant un dispositif de thérapie expiratoire positive et un raccord de sortie (100) selon l'une quelconque des revendications 1 à 12.

14. Dispositif de thérapie expiratoire positive comprenant le raccord de sortie (100) selon l'une quelconque des revendications 1 à 12.

15. Dispositif de thérapie expiratoire positive selon la revendication 14, ledit raccord de sortie (100) étant intégré au dispositif de thérapie expiratoire positive.
